# EUROPEAN PATENT SPECIFICATION

(11) **EP 3 316 223 B1**
(45) Date of publication and mention of the grant of the patent: **16.10.2019**
(21) Application number: 17198497.4
(22) Date of filing: 26.10.2017
(51) Int. Cl.: G06T 19/20, G06F 3/048

(54) **VISUALIZATION SYSTEM AND METHOD FOR 3D SCENES**
VISUALISIERUNGSSYSTEM UND -VERFAHREN FÜR 3D-SZENEN
SYSTÈME ET PROCÉDÉ DE VISUALISATION DE SCÈNES 3D

(30) Priority: 31.10.2016 GB 201618341
(43) Date of publication of application: 02.05.2018
(73) Proprietor: Bitplane AG, 8048 Zürich (CH)
(72) Inventor: MAJER, Peter, 8048 Zurich (CH)
(74) Representative: FRKelly

(56) References cited:
- EP-A1- 1 063 617
- US-B2- 6 847 348
- CIGNONI P ET AL: "MAGICSPHERE: AN INSIGHT TOOL FOR 3D DATA VISUALIZATION", COMPUTER GRAPHICS F, WILEY-BLACKWELL PUBLISHING LTD, GB, vol. 13, no. 3, 1994, pages 317-328, XP008035104, ISSN: 0167-7055, DOI: 10.1111/1467-8659.1330317

## Description

### Field of the Invention

The present invention relates to computer-implemented visualization of three dimensional (3D) scenes in the context of computer drawing tools.

### Background of the Invention

In a computer system for visualization of three dimensional (3D) scenes, data representing a 3D scene is projected onto a camera's view projection plane from a camera's defined viewpoint and mapped onto a two-dimensional computer display. This computer rendering procedure usually causes some parts of the scene to be occluded by other parts of the scene. Such occlusions reduce the clarity of the visualization to a user, which can be particularly problematic if the user is attempting to use a computer drawing tool to create a drawing within the 3D scene or to select a region of the scene for further processing. It would be desirable to mitigate the problems outlined above.

An article entitled "MagicSphere: an insight tool for 3D data visualization" by Cignoni et al (Computer Graphics F, Wiley-Blackwell Publishing Ltd., GB, vol. 13, no. 3, 1994, pages 317-328, XP008035104, ISSN:0167-7055, DOI:10.1111/1467-8659.1330317)discloses a tool for the visual analysis of 3D datasets or geometries, which allows interactive focusing on regions of interest using a 3D logic input device.

Patent document US2006227130 discloses agraphical method and system to create one-dimensional, two-dimensional, and/or three-dimensional drawings of designs directly in 3D on a computer monitor screen.

### Summary of the Invention

A first aspect of the invention provides a visualization method as claimed in claim 1. A second aspect of the invention provides a visualization system as claimed in claim 15. Preferred features of the invention are recited in the dependent claims.

Advantageously, the method includes causing any part of said 3D drawing structure within said highlight region to be highlighted when rendered on said display device in said 2D image. Optionally, the method includes causing all of said 3D drawing structure to be highlighted when rendered on said display device in said 2D image.

The method may include defining a second highlight region in said 3D scene with respect to said 3D pointer location or with respect to said 3D drawing structure, and causing any part of said 3D drawing structure, and optionally any 3D scene data, within said second highlight region to be highlighted when rendered on said display device in said 2D image.

The method may include generating said 3D drawing structure data from at least two 3D pointer locations corresponding to movement of said pointer with respect to said 3D scene. Optionally the method includes defining a 3D line in said 3D scene from said 3D drawing structure data. Said 3D line may be rendered in said 2D image. Said 3D line may have a two dimensional transverse cross-section.

Optionally, the method includes generating said 3D drawing structure data and causing a 3D drawing structure corresponding to said 3D drawing structure data to be rendered in said 2D image during movement of said pointer, the method further including causing said highlight region to track said movement of said pointer.

In cases where the second highlight region is defined, the method may include causing said second highlight region to track said movement of said pointer.

Said 3D drawing structure may comprise a 3D line, and said method may include causing said highlight region to track said 3D line.

In cases where the second highlight region is defined, the method may include causing said second highlight region to track said 3D line.

The preferred method includes, in response to detecting a change in said 3D pointer location with respect to said 3D scene, determining a new 3D location of a pointer with respect to said 3D scene; defining a new highlight region in said 3D scene with respect to said new 3D pointer location; determining which of said 3D scene data is within said new highlight region; and causing the 3D scene data within said highlight region to be highlighted when rendered on said display device in said 2D image.

The method preferably includes causing said highlight region to track said movement of said pointer.

Optionally, the method includes causing at least part, and preferably substantially all of, said highlight region to be in front of said 3D pointer location in the direction of movement of said pointer.

The method may include causing said highlight region to be elongate, for example ellipsoidal, and preferably aligned with a direction of movement of the pointer.

The method may include locating a 2D pointer on the rendered 2D image and calculating said 3D pointer location from the location of said 2D pointer and said 3D scene data. Alternatively, the method may include locating a 3D pointer in the rendered 3D scene.

Optionally, the method includes analysing the 3D scene data with respect to one or more 3D pointer location, identifying one or more 3D structure in the relevant 3D scene data and generating 3D drawing structure data corresponding to the, or each, identified 3D structure.

The method may include defining a 3D selection structure from at least one of said 3D pointer locations and determining which of said 3D scene data corresponds with said 3D selection structure. Defining said 3D selection structure may involve generating 3D selection structure data from said at least one 3D pointer location. The method may include performing one or more processes on the 3D scene data corresponding to said 3D selection structure data. Said 3D selection structure may be a 3D drawing structure. Said 3D selection structure data may comprise 3D drawing structure data.

In use, the preferred visualization system projects data representing a three dimensional (3D) scene onto a camera's view projection plane from a camera's defined viewpoint and maps and renders the projected scene onto a two-dimensional computer display. In the rendered image some parts of the scene may be occluded by other parts of the scene. When the user positions a 3D pointer in the scene, a region of the scene around the 3D pointer is highlighted, e.g. by rendering the rest of the scene with increased transparency and/or reduced intensity. This enables improved visualization of the highlighted region when it is occluded by other parts of the scene because the contribution of these other parts to the rendering is reduced. This improved visualization around the 3D pointer is particularly useful when the 3D pointer is also used for drawing a 3D structure into the scene as the highlight allows the user to perceive the local region around the pointer and to understand where to continue drawing in 3D. The combination of using the same 3D pointer to draw a 3D structure and to highlight a region around the 3D pointer provides a highlighter to the drawing tool with which the user traces a drawing in 3D that may otherwise be occluded by parts of the 3D scene that are in the line of sight between the camera and the 3D pointer. The combination of using the same 3D pointer to draw a 3D structure and to highlight a region around the 3D pointer can significantly aid the users' ability to perform the drawing . Hence, preferred embodiments of the invention provide highlighting of portions of a computer rendered 3D scene to disambiguate occlusions in the rendered image, especially while a user is drawing using a pointer device.

The 3D pointer may be positioned in the scene using a 3D pointing device or by positioning a 2D pointer on the 2D image rendered on the display and calculating a 3D location from the location of the 2D pointer. In either case the positioning is facilitated by visual feedback of the pointer being rendered to the computer displayed image and by the highlighting around the 3D location.

Embodiments of the invention may include or be used in conjunction with a computer drawing tool whereby a 3D drawing may be created in the 3D scene using one or more 3D pointer locations.

Further advantageous aspects of the invention will be apparent to those ordinarily skilled in the art upon review of the following description of specific embodiments and with reference to the accompanying drawings.

### Brief Description of the Drawings

Embodiments of the invention are now described by way of example and with reference to the accompanying drawings in which like numerals are used to denote like parts and in which:
Figure 1 is a schematic diagram of a computer-implemented visualization system embodying one aspect of the invention;
Figure 2 is a schematic plan view of a 3D scene, a projection plane and a viewpoint;
Figure 3A is a schematic plan view of the 3D scene of Figure 2 including a pointer and a highlight region for a first pointer position;
Figure 3B is a schematic plan view of the 3D scene of Figure 2 including a pointer and a highlight region for a second pointer position;
Figure 3C is a schematic plan view of the 3D scene of Figure 2 including a pointer and a highlight region for a third pointer position;
Figure 4 is a flow chart illustrating a computer-implemented visualization method embodying another aspect of the invention;
Figures 5A to 5D each shows a computer rendered 2D image of a 3D scene with a respective portion of the image highlighted;
Figure 6 is a flow chart illustrating a preferred computer-implemented visualization method embodying the invention with drawing functionality;
Figures 7A to 7D each shows a computer rendered 2D image of a 3D scene with a respective portion of the image highlighted and showing the progression of a drawing of the image;
Figure 8 is a flow chart illustrating a highlighting method for use with embodiments of the invention; and
Figure 9 is a schematic diagram of an alternative embodiment computer-implemented visualization system including a drawing tool.

### Detailed Description of the Drawings

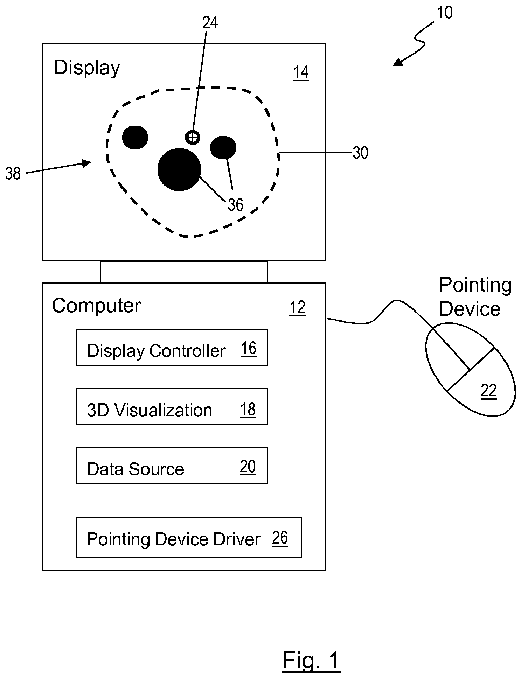

Referring now to Figure 1 of the drawings there is shown, generally indicated as 10, a system for visualizing data representing a 3D scene, the system being an exemplary embodiment of one aspect of the present invention. Typically, the system 10 comprises a computer 12 and a display device 14. The display device 14 is conveniently a computer monitor but may alternatively be any display device capable of receiving image data from a computer or other electronic device and displaying corresponding 2D images. The system 10 further includes a display controller 16, for example being of the type commonly referred to as a video display controller or a graphics controller, for controlling the display of images on the display device 14. The system 10 includes a 3D visualization module 18 for processing data received from a data source 20 and generating processed data for display on the display on the display device 14 via the display controller 16. In the illustrated example it is assumed that the 3D visualization module 18 controls the rendering of images on the display device 14 via the display controller 16. The 3D visualization module 18 may be implemented in software, firmware and/or hardware as appropriate, and may for example include any combination of a data processor, graphics processor and software application(s) for performing the tasks described herein, The data source 20 may take any suitable form, for example data stored on a computer memory. The data source 20 may alternatively, or in addition, comprise (or be connected to) a data capturing device, e.g. a camera or microscope. The data capturing device may form part of the system 10 or may be connectable to the system 10 during use or off-line.

The system 10 includes a pointing device 22 for allowing pointing actions of a user (not shown) to be translated into corresponding movement of an on-screen pointer 24 on the display device 14. The pointing device 22 and pointer 24 are parts of a user interface with the computer 12. The pointing device 22 may comprise a computer mouse (as illustrated), a trackball, a touch screen, a touch pad, a keyboard or any other suitable known input device. The pointing device 22 and pointer 24 are supported by a pointing device driver 26. The pointer 24 is a 3D pointer. In the illustrated embodiment, the 3D pointer 24 is implemented as a 2D on-screen pointer combined with 2D to 3D pointer location mapping, which is conveniently performed by the pointing device driver 26. The 2D to 3D mapping involves determining a 3D location in the displayed 3D scene corresponding to the 2D location of the pointer 24 on the display 14. As such the 2D location of the pointer 24 on the screen of the display device 14 is given a 3D location with respect to the 3D scene that is rendered by the display 14 as a 2D image. In the present case this means that the on-screen 2D location of the pointer 24 is mapped to a 3D location in the 3D scene that is being visualized by the system 10. In this case, the pointing device 22 is a 2D pointing device controlling a 2D on-screen pointer, but the 2D to 3D pointer location mapping causes the pointer 24 to operate as a 3D pointer. The 2D to 3D pointer location mapping which causes the pointer 24 to operate as a 3D pointer may be implemented by an optimization method that searches for the best 3D pointer location in the line of sight through the 2D pointer location. In the case where the 3D pointer is used for drawing, the method may involve using information about previous 3D pointer positions to improve continuity of the drawing. In alternative embodiments, the pointing device 22 may be a 3D pointing device that controls a 3D pointer. In such cases there is no need for 2D to 3D pointer location mapping since the 3D pointer 24 is generated and controlled in 3D with respect to the displayed 3D image. By way of example, 3D pointing devices are available from Leap Motion Inc. of San Francisco, USA.

In the illustrated embodiment, the display controller 16, 3D visualization module 18 and pointing device driver 26 are assumed to be components of the computer 12. The data source 20 may comprise a computer memory on the computer 12 storing data provided in any convenient way, for example received off-line from any suitable external system, or received in real-time or off-line from a data capturing device which may be part of the system 10 or connectable to the system 10. In cases where the data is received in real-time, the data source 20 may comprise a buffer memory.

More generally, the system 10 comprises one or more computer programs supported by one or more computers or other electronic hardware devices, as is convenient and as would be apparent to a skilled person. The components (hardware and software) of the system 10 may be centralized (as illustrated) or distributed as is convenient. Any distributed components may communicate with the other components as required by any convenient conventional computer network or other computer communication means, e.g. LAN, WAN, internet, Ethernet, or as a peripheral computing device.

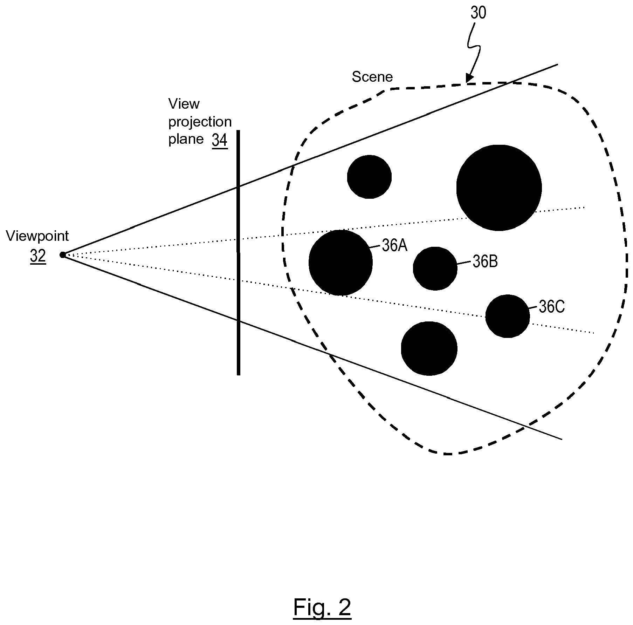

Figure 2 shows a plan view of a 3D scene 30, a viewpoint 32 for the scene 30 and a two dimensional (2D) projection plane 34 onto which the 3D scene may be projected in two dimensions (2D). For the purposes of example only the 3D scene comprises a plurality of objects 36 that are spaced apart in three dimensions. When the 3D scene is projected onto the projection plane 34 with respect to viewpoint 32 object 36B is completely occluded by object 36A, and object 36C is partially occluded by object 36A.

For a computer visualization of the scene 30, data representing 3D scene (which may be referred to as 3D image data) is projected onto the 2D projection plane 34 to produce corresponding 2D image data, i.e. data that represents the 2D projection of the 3D scene onto the projection plane 34. Any conventional 3D to 2D projection method may be used for this purpose, for example orthographic projection or perspective projection. The 2D image data may then be rendered on the display device 14. In the example of Figure 1, the 3D image data is provided from the data source 20 to the 3D visualization module 18, which performs 3D to 2D projection on the 3D image data to produce corresponding 2D image data. The 2D image data is provided to the display controller 16, which causes a corresponding 2D image 38 of the 3D scene 30 to be rendered on the display device 14. The 3D image data is typically captured by a camera, microscope or other data capture device (not shown) and provided to the data source 20 in real time or off-line. Alternatively, the 3D image data may be computer generated and provided to the data source 20 in real time or off-line.

Typical 3D image data could be described as falling into two categories: a) geometric data with clear/sharp boundaries. A sphere, a cube, a cone and of course more complex data are examples of this type; b) 3D volume images where each point in space has an intensity. Even if the image depicts an object like a sphere or a neuron or some other structure it is not clearly defined where that structure is. 3D volume images may also be described as 3D volumetric images or 3D scalar field images. In any event embodiments of the invention are particularly but not exclusively suited for use with 3D volume images.

Geometry data may be rendered e.g. with shading, reflections, shadows, or other effects. Volume image data may be rendered typically but not necessarily by maximum intensity projection or other direct volume rendering techniques using any type of transfer function to enhance certain characteristics of the data.

The 3D visualization module 18 therefore typically includes functions for projecting the three-dimensional objects included in the three-dimensional scene onto the camera's view projection plane 34 as seen from the camera's defined viewpoint 32. By mapping the two-dimensional image from the view projection plane 34 through the display controller 16 onto the two-dimensional display 14, the two-dimensional image 38 of the three-dimensional scene 30 is displayed on the display 14. It will be understood that the role of the display controller 16 in the above tasks can vary depending on the configuration of the system and on the capabilities of the controller 16. For example it is possible that some of the 3D to 2D projection is performed by the display controller 16.

Typically, the data provided by the data source 20 defines the 3D scene 30 on the basis of one or more three-dimensional objects 36, each object 36 being defined by its shape, size, and location. The source data could be 3D image data such as provided by a microscope or other 3D imaging device, e.g. a computer tomography device or magnetic resonance imaging device.

Conveniently, the functionality of the 3D visualization module 18 with regard to 3D visualization is provided by using one or more application programming interface API. For example, the 3D visualization module 18 may include program functions from OpenGL, by Silicon Graphics, Inc., or from Direct3D from Microsoft, Inc., or from Vulkan by Khronos Group Inc.

In any event, when the 2D image 38 is viewed by a user on the screen of the display device 14, some objects of the 3D scene may be wholly or partly occluded by other objects of the scene. To mitigate the occlusion problem, in the 2D image 38 all or part of one or more objects of the 3D scene 30 are highlighted with respect to at least some and preferably all of the remainder of the 3D scene 30 depending on the location of the pointer 24 with respect to the 3D scene 30. To this end, a 3D location of the pointer 24 with respect to the 3D scene 30 is determined and a highlight region 40 is defined with respect to the determined location of the pointer 24. The highlight region 40 is a 3D region around, e.g. centred on, or otherwise defined with respect to the pointer 24 location with respect to the 3D scene. The highlight region 40 may take any desired shape and/or size. For example it may be spherical or ellipsoidal. Preferably the system 10 is configured to support user adjustment of the shape and/or size of the highlight region.

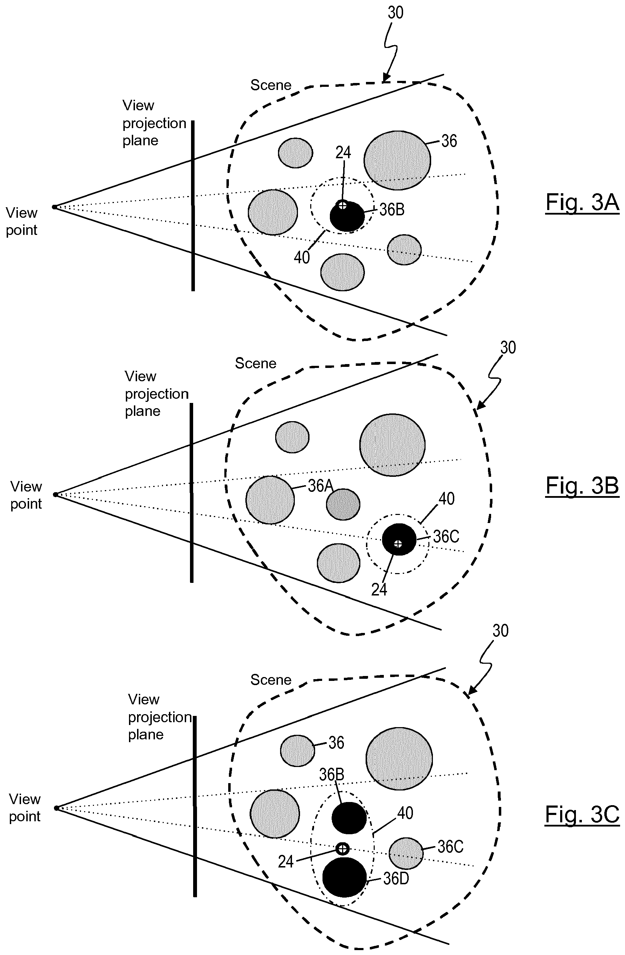

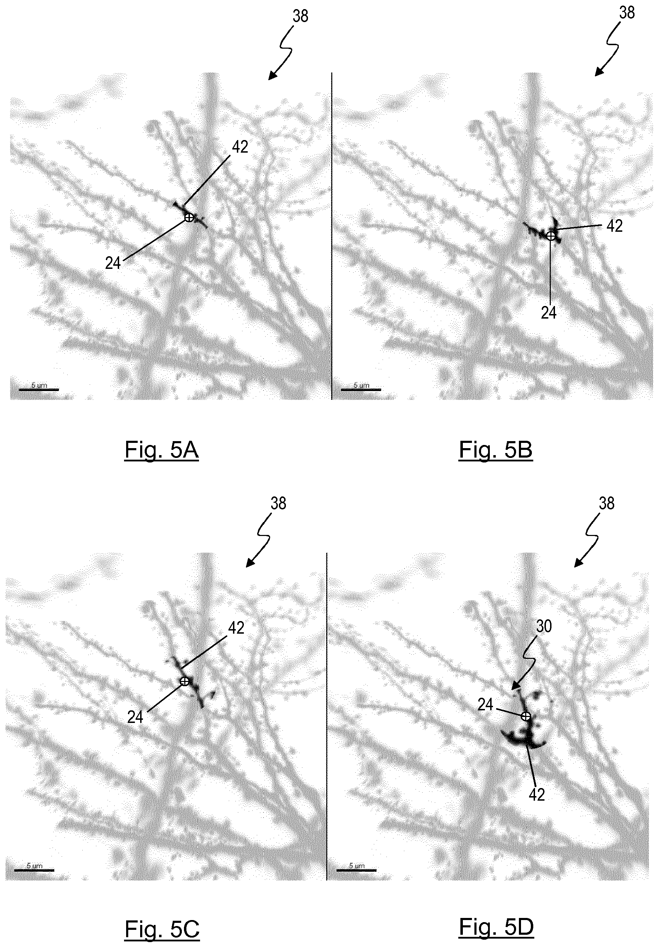

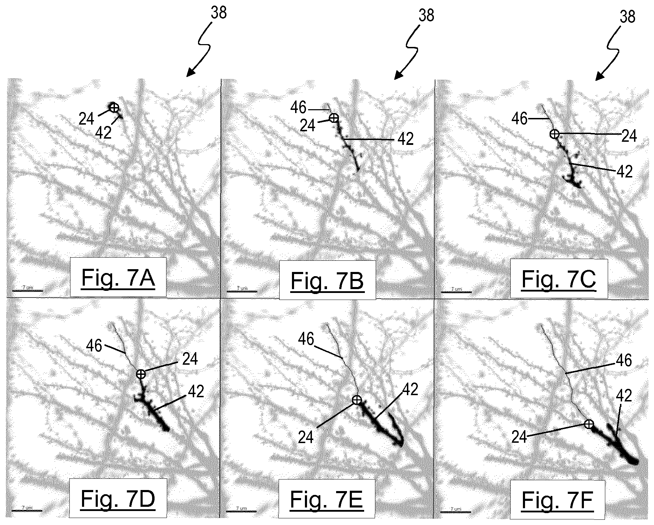

The effect of the highlighting method is illustrated in Figures 3A to 3C. In Figure 3A, the pointer 24 is located close to object 36B such that object 36B is entirely within the highlight region 40. None of the other objects 36 overlap with the highlight region 40. Therefore, object 36B is highlighted with respect to the other objects 36 of the scene. In Figure 3B, the pointer 24 is located close to object 36C such that object 36C is entirely within the highlight region 40. None of the other objects 36 overlap with the highlight region 40. Therefore, object 36C is highlighted with respect to the other objects 36 of the scene. In Figure 3C, the pointer 24 is located close to objects 36B and 36D such that objects 36B and 36D are entirely within the highlight region 40. None of the other objects 36 overlap with the highlight region 40. Therefore, objects 36B and 36D are highlighted with respect to the other objects 36 of the scene. It is noted that the objects in the scene are not necessarily geometrically defined objects. The objects may be objects within a 3D volume image. Examples of such objects are depicted in Figures 5 and 7 in the form of neuron structures.

The highlighting may be effected by any means that provides a visual distinction in the rendered 2D image 38 between highlighted and non-highlighted parts of the image. For example, the highlighting may involve adjusting any one or more visual rendering characteristic, including for example the intensity, opacity, transparency, colour or form (e.g. broken or solid lines), of the highlighted parts and/or non-highlighted parts of the image such that the highlighted parts are visually discernible to the user. For example, in Figure 3A, object 36B is visible to the user viewing image 38 because the contribution to the projection of the objects outside of the highlight region is reduced (absolutely or relatively depending on which highlighting technique(s) are used).

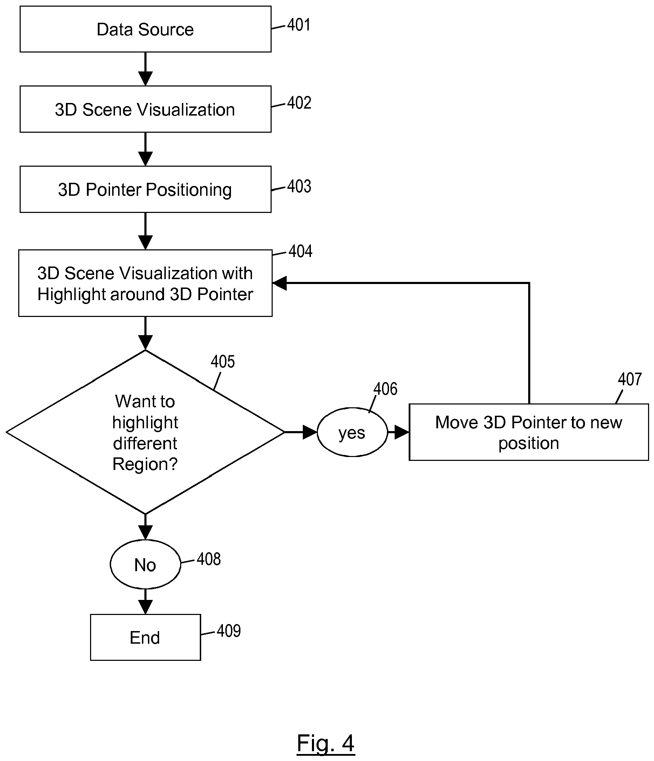

Figure 4 is a flow chart illustrating an embodiment of the computer-implemented visualization method. The 3D image data is provided to the 3D visualization module 18 from the data source 20 (401, 402). The 3D visualization module 18 performs 3D to 2D image projection on the 3D image data to produce corresponding 2D image data, and the 2D image data is rendered on the display device 14 as a 2D image 38 (402). Then the user positions the 3D pointer 24 to a desired location in the displayed 3D scene such that the highlight can be rendered on this part of the scene (403). In the present embodiment this involves the pointer 24 being positioned by the user at a desired location on the rendered image 38 and a corresponding 3D location with respect to the 3D scene 30 being determined (403).

At 404, the 3D visualization module 18 obtains data indicating the 3D location of the pointer in the 3D scene 30 and determines the corresponding highlight region 40. The 3D visualization module 18 compares the 3D image data with the highlight region 40 and determines which element(s) of the 3D image data are to be highlighted. The 3D visualization module 18 then causes the relevant 3D image elements to be highlighted when the 3D image data is rendered on the display device 14. To this end, the 3D image data and data defining the highlight region 40 may be passed to the part of the visualization module 18 (and/or display controller 16 depending as applicable) that renders the image 38 so that the relevant portion(s) of the 3D scene within the highlight region 40 are rendered in a highlighted manner. The manner of the highlighting may be determined by the relevant rendering characteristic(s).

If the user changes the 3D pointer location this causes the 3D visualization module 18 to cause part(s) of the rendered image 38 to be highlighted (405, 406, 407, 404) corresponding to the new 3D pointer location. Otherwise, the process ends (408, 409). Accordingly the highlighted part(s) of the rendered image, and more particularly the highlighted part(s) of the 3D scene track the movement of the pointer 24. In the present embodiment, changing the 3D pointer location involves the user operating the pointing device 22 to move the location of the on-screen 2D pointer 24 after which a corresponding new 3D pointer location is calculated. In alternative embodiments it simply involves operating a 3D pointing device.

Typically, the visualization module 18, together with the display controller 16, refreshes the displayed image, including any required highlighting, in response to any action, in particular a user action, that requires the displayed image to be changed. Such actions include moving the pointer 24 or manipulating the image (e.g. to zoom or change viewpoint).

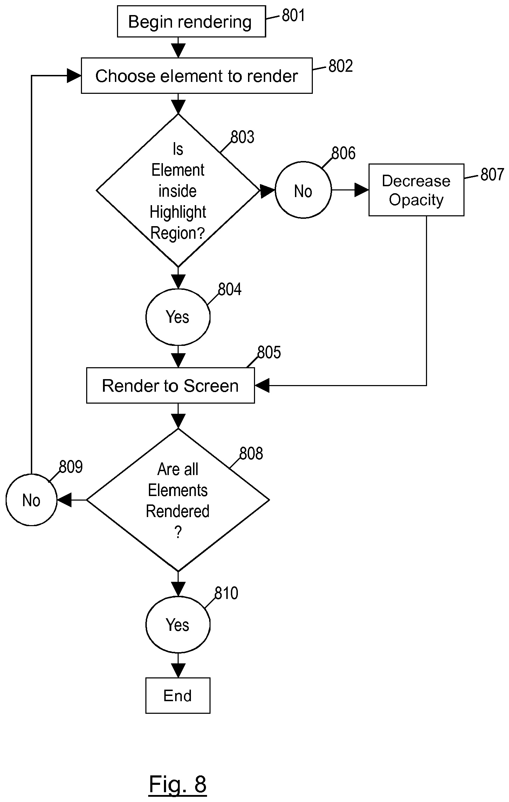

The visualization module 18 applies the required highlighting to the 3D data representing the 3D scene 30. Figure 8 illustrates an example of how the 3D visualization module 18 may apply highlighting to the 3D data. At 801 the image rendering process begins. At 802, the 3D visualization module 18 selects an element of the image data to render. In general, the 3D scene 30 may comprise one or more objects, and the corresponding data may be rendered by the system 10 in data elements, where each data element may include data representing all or part of one or more of the objects of the scene. The objects may be defined by geometric data or by volume image data depending on the nature of the data. At 803, the 3D visualization module 18 calculates if the selected data element is within the highlight region 40 (as is currently defined depending on the 3D location of the 3D pointer 24). In this example, it is assumed that highlighting is applied to the relevant data elements by reducing the opacity of the non-highlighted data elements. Other visualization changes that result in a highlighting effect could be used instead of, or in addition to, changes in opacity. Therefore, if it is determined that a data element is within the highlight region 40, it is simply rendered to the screen of the display device 24 (804, 805), e.g. using default visual rendering characteristics. However, if it is determined that a data element is not within the highlight region 40 it is rendered to the screen of the display device with a reduced opacity (e.g. in comparison with the default opacity) (806, 807, 805). Steps 802 to 807 are repeated until all data elements are rendered, after which the rendering process is finished (808, 809, 810). The result is that the data elements that are determined to be within the highlight region, and therefore the corresponding object(s) or part-object(s) in the data scene 30, are rendered to the user in a highlighted manner with respect to the other data elements, and therefore the rest of the scene 30.

Figures 5A to 5D each shows an example of the rendered 2D image 38 illustrating the effect of the highlighting described with reference to Figures 4 and 8. In Figures 5A to 5D the 3D scene being depicted comprises neuron structures. In each figure, the pointer 24 has a different location in the 3D scene 30 and a corresponding different part 42 of the 3D scene 30 is highlighted in the image 38. In this example, the non-highlighted parts of the 3D scene are rendered with a decreased opacity in comparison with the highlighted part 42. The highlighted parts 38 are therefore visually discernible by the user whereas, without the highlighting, they would have been obscured by other parts of the 3D scene 30 displayed in the image 38.

More generally, the system 10 may support a first set of one or more visual rendering characteristics for use with parts of the data representing the 3D scene that are to be highlighted, and a second set of one or more visual rendering characteristics for use with parts of the data that are not to be highlighted, the first set differing from the second set in any way that causes the highlighted parts to be visually discernible by the user from the non-highlighted parts when the image 38 is rendered on the display 14. The visual rendering characteristics typically include any one or more of intensity, opacity, transparency, colour or form.

In alternative embodiments, it is not essential that all of the non-highlighted parts of the image 38 are rendered differently than the highlighted part(s) so long as at least a region around the highlighted part(s) is rendered differently to allow the user to clearly see the highlighter part(s).

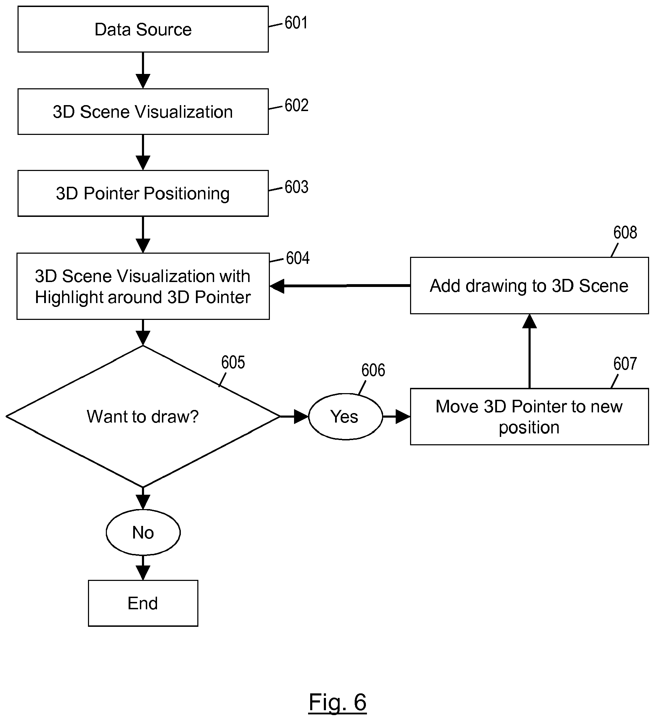

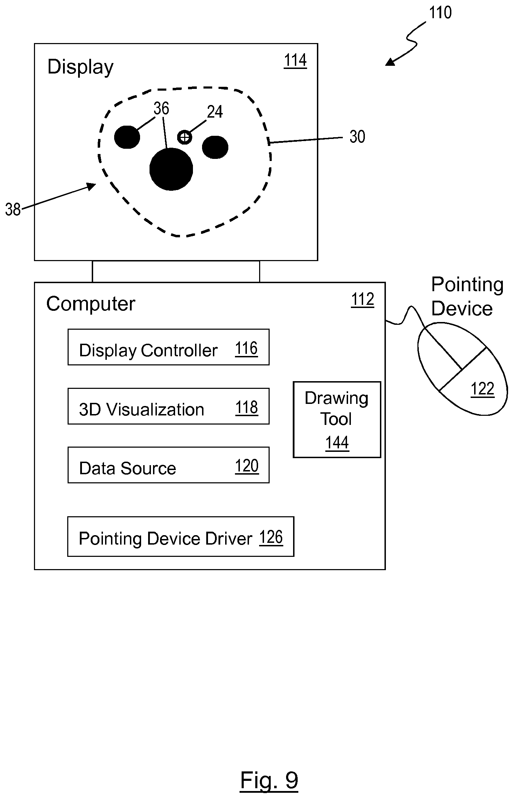

Referring now in particular to Figures 6, 7A to 7F and 9, the highlighting method described above is used in conjunction with a computer drawing tool. In addition to the display device 114, display controller 116, image processor 118, data source 120, pointing device 122 and pointing device driver 126 (any or all of which may be the same or similar to those described with reference to Figure 1) the system 110 includes a computer drawing tool 144, for example a drawing editor. The drawing tool 144 allows the user to create a drawing in the displayed 3D scene 30 using the pointing device 122. Even though the drawing is displayed by the display device in 2D, it is a 3D drawing in that it is defined by 3D data with respect to the 3D scene. The drawing tool 144 may use the 3D pointer locations within the 3D scene to create a 3D drawing. For example, moving the pointer 24 from one 3D location to another in the 3D scene may create a 3D drawing line between the two 3D locations. The drawing may therefore be part of the 3D scene 30. Advantageously, the highlighting described above is performed simultaneously with the drawing. The same pointer 24 is used for both drawing and highlighting. The region of the displayed 3D scene 30 on which the user is drawing is highlighted such that the corresponding part(s) of the 3D scene can more clearly be seen, as described above. As such, the highlighting tracks the drawing. This facilitates tracing parts of the 3D scene 30 in cases where those parts may otherwise be wholly or partly occluded.

More generally, the invention allows the user to draw a 3D drawing structure (e.g. a 3D line or 3D shape (regular or irregular) into the 3D scene 30 using at least one but typically more than one of the recorded 3D pointer locations. The, or each, 3D pointer location may be used to create the drawing directly (e.g. by joining pointer locations to create a line) or to aid the creation of the drawing (e.g. one or more 3D pointer locations may be used in conjunction with one or more algorithm, for example a segmentation or curve-fitting algorithm, to create the drawing). The system 110 highlights the computer rendered 3D scene in the region where the user is currently drawing and suppresses visualization of other parts of the scene allowing the user to better see the drawing and the 3D scene in the region of drawing. Typically the displayed 3D scene initially contains a volume image. After the user has drawn the displayed 3D scene comprise the volume image and the 3D geometry of the drawing. In the context of this invention, the term 3D line is intended to embrace not only lines that have no thickness but also those that are two dimensional in transverse cross-section, e.g. tube-like or cylinder-like in structure, or with any other desired transverse cross sectional shape. The 3D lines may be straight, curved or curvilinear as required. Creating drawings in the form of 3D lines is particularly useful in cases where the 3D scene depicts elongate objects such as fibres, filaments or blood vessels. Alternatively, the 3D structure may be a sphere, or other 3D shape, which is particularly useful in cases where the 3D scene depicts spots, spheres or the like.

Typically, the drawing tool 144 receives from the pointer driver 126 the 3D pointer locations, records them and constructs a 3D drawing from the recorded data. The drawing tool 144 typically also accesses the 3D image data to precisely position the drawing in relation to the content of the 3D image. The 3D drawing is added to the 3D scene 30, conveniently by the drawing tool 144. In any event the 3D drawing data is provided to the visualization module 18 and is rendered by the visualization module 18 and display controller 16, as part of the 3D scene, including any relevant highlighting. Drawing methods with a higher degree of automation may use one or more of the recorded 3D pointer locations as an input to a computer implemented algorithm that computes the drawing using the data defining the 3D scene 30. For example a segmentation algorithm may analyse the 3D scene data with respect to one or more 3D pointer location, identify one or more 3D structure in the relevant 3D scene data and create one or more 3D drawing structure corresponding to the identified 3D structure(s). Creation of the 3D drawing structure(s) may be done using the 3D scene data.

Figure 6 is a flow chart illustrating an embodiment of the computer-implemented visualization method including drawing functionality. Steps 601 to 604 are in this example the same as steps 401 to 404, respectively, shown and described in relation to Figure 4. In steps 605 to 608, if the user wishes to draw all or part of the displayed 3D scene, beginning with the 3D pointer at the desired starting location in the 3D scene, the 3D pointer is moved by the user to trace all or part of the 3D scene during which the corresponding 3D drawing is created as described above and added to the 3D scene so that it is rendered as part of the 3D scene. Advantageously, the highlighting described above, for example with reference to steps 405, 406, 407, 404, is simultaneously performed so that the portion of the 3D scene that the user is drawing is highlighted to facilitate drawing. Hence the 3D pointer for drawing is combined with the highlighting function, i.e. drawing and highlighting using the same pointer.

Figures 7A to 7F each shows an example of the rendered 2D image 38 illustrating the effect of highlighting in combination with drawing. In Figures 7A to 7F the 3D scene being depicted comprises neuron structures and is a volume image. In each figure, the pointer 24 has a different location on the image 38 and a corresponding different part 42 of the3D scene 30 is highlighted. In this example, the non-highlighted parts of the 3D scene 30 are rendered with a decreased opacity in comparison with the highlighted part 42. The highlighted parts are therefore visually discernible by the user whereas, without the highlighting, they would have been obscured by other parts of the 3D scene 30. Figure 7A shows a start location for the pointer 24 and in Figures 7B to 7E the pointer 24 is shown moving along the highlighted neuron structure until it reaches an end location in Figure 7F. In addition to highlighting the neuron structure, this movement of the pointer 24 creates a drawing line 46 in the 3D scene 30 between the start and end locations. The combination of using the same 3D pointer location for drawing and for highlighting around this location facilitates drawing along the line of interest, especially in situations where parts of the 3D scene close to the drawing are occluded by other parts of the 3D scene.

When drawing a line, it is advantageous that the highlight region 40 is elongate (e.g. ellipsoidal) and aligned with the direction of movement of the 3D pointer 24 with respect to the 3D scene such that the region of the image in front of the moving 3D pointer 24 is highlighted. For example, instead of being centred around the pointer location, the highlight region 40 may be positioned so that the 3D pointer 24 location is at or near the rear of the highlight region 40. The preferred arrangement is such that substantially all of the highlight region 40 extends forwardly of the pointer location in the direction of movement of the 3D pointer 24, or at least such that relatively less of the highlight region extends rearwardly and laterally of the pointer location.

In some embodiments the 3D drawing structure, when rendered to the image 38, may be unhighlighted. Alternatively, the 3D visualization module 18 may be configured to highlight part of, or the whole of, the rendered 3D drawing structure. For example, the 3D visualization module 18 may be configured to highlight any part of the 3D drawing structure that is within the highlight region 40. This may conveniently be achieved by including the 3D drawing structure data in the 3D scene data. Alternatively a second highlight region (not illustrated) may be defined and the 3D visualization module 18 may be configured to highlight any part of the drawing structure, and optionally also the 3D scene, that is within the second highlight region. The rendering characteristic(s) used to create the highlighting in the second region may be different from the highlight rendering characteristic(s) for the first region 40. The second highlight region may be defined in the same manner as the highlight region 40 (or may be defined with respect to the drawing structure) but typically has a size, shape and/or location with respect to the pointer 24 that is different than that of the highlight region 40. For example the second highlight region may be configured such that highlighting extends forwards and rearwards along the drawing with respect to the pointer location, or just rearwards with respect to the pointer location.

Optionally, the 3D pointer 24 may be used to select one or more parts of the 3D scene. For example, in a selecting mode of operation, one or more 3D pointer locations in the 3D scene are recorded and used to define one or more selected parts of the 3D scene. The, or each, selected scene part may for example be a 3D line, a 3D shape or other 3D structure as defined by the respective 3D pointer location(s) for the purpose of selection. For example a selected 3D line may be defined by two or more pointer locations while a selected 3D shape, e.g. a sphere, may be defined by one or more 3D pointer locations. Once the, or each, part of the 3D scene is selected, the corresponding part(s) of the 3D scene data are determined. This may be performed by comparing the 3D scene data against data defining the, or each 3D selection structure created by the selection process and determining which of the 3D scene data are within the, or each 3D selection structure. The 3D selection structure data may comprise the relevant pointer location(s) and/or data derived from the pointer location(s). Determination of the selected 3D scene data is conveniently performed by the 3D visualization module 18, although a selection module (not shown) may alternatively be provided for this purpose. For example, in selecting mode, the user positions the 3D pointer in the 3D scene at one or more 3D locations in the 3D scene to define one or more selected parts of the 3D scene. The 3D visualisation module 18 (or other selection module) receives from the pointer driver 126 the 3D pointer location(s), records them and constructs one or more 3D selection structure from the recorded data. The corresponding 3D scene data can then be determined. The selected 3D scene data may then be used for any desired processing purpose(s), e.g. edge detection or de-noising. The 3D visualisation module 18 may be configured to perform such processing and/or the selected 3D scene data may be communicated to one or more other processing device (not illustrated) for further processing,

In some embodiments, the selection of parts of the 3D scene may be performed in conjunction with the 3D drawing described above such that the, or each, 3D drawing structure also serves as the 3D selection structure. Accordingly, the 3D selection structure may be added to the rendered 3D scene in the form of the 3D drawing structure. In such embodiments the 3D drawing module 144 may be configured to create the 3D selection/drawing structure and/or determine which of the 3D scene data are within the, or each 3D selection structure, as is convenient. Advantageously, however, the 3D selection process is in any event performed in conjunction with the 3D highlighting described above.

The pointing device 22, 122 makes it possible for the user to provide to the system 10, 110 signals indicative of pointer location. Preferably the pointing device 22, 122 is provided with means for providing to the system 10, 110 not only pointer location signals for positioning the pointer 24 in the two dimensions of display, but also in the depth dimension vertical to the two dimensions of the display (and may therefore be described as a 3D pointing device). If the pointing device 22, 122 itself does not provide a depth signal (i.e. it is a 2D pointing device), the driver 26, 126 may generate such a signal from the 2-dimensional signal provided by the pointing device 22, 122. In one embodiment the depth parameter may be calculated from the 3D scene by selecting the depth position calculated to be an optimal depth position of all possible values along the line of view corresponding to the 2-dimensional positioning provided by the pointing device. However, there are many different ways in which a 3D location can be calculated from a 2D pointing device signal and the 3D scene. One example is described in the publication "Virtual finger boosts three-dimensional imaging and microsurgery as well as terabyte volume image visualization and analysis" by Peng et al., Nature Communications 5, Article no. 4342, doi:10.1038/ncomms5342, 11 July 2014. Determining useful 3D pointer locations from user input may include repositioning the pointer in 3D (both depth and screen coordinates) to make it automatically snap to certain structures in the 3D scene. In particular when using the pointer for drawing this automatic repositioning can help the user. In any event, as a result the pointing device 22, 122 with the pointing device driver 26, 126 makes it possible for a user to position a 3D pointer in the 3D scene.

It will be seen from the foregoing that to implement a highlight around the 3D pointer 24 the 3D pointer position is passed to and used by the 3D visualization module 18, 118 to render those parts of the 3D scene that are within a highlight region 40 around the 3D pointer differently from those parts that are outside this region. The region 40 can be spherical or ellipsoidal or a 3D box or it can be an arbitrarily shaped region. In one embodiment the 3D visualization module 18, 118 computes for each element of the 3D scene its distance to the 3D pointer and renders elements that are within a given distance differently from those objects that are outside the given distance. For example the elements outside the highlight region may be rendered with lower opacity.

For performance reasons the 3D visualization module 18, 118 may be implemented on the Graphics Processing Unit of a computer. Typically a fragment shader program could be used to efficiently implement a 3D highlight of a volume image. Similarly a vertex shader program could be used to efficiently implement a 3D highlight of objects rendered by a vertex shader.

It will be understood that methods embodying the invention may be implemented in software, firmware, hardware, or a combination thereof. In one mode, the method is implemented in software, as one or more executable program, and is executed by one or more special or general purpose digital computer(s), such as a personal computer (PC; IBM-compatible, Apple-compatible, or otherwise), personal digital assistant, workstation, minicomputer, or mainframe computer. The steps of the method may be implemented by a server or computer in which the software modules reside or partially reside.

Generally, in terms of hardware architecture, such a computer will include, as will be well understood by the person skilled in the art, a processor, memory, and one or more input and/or output (I/O) devices (or peripherals) that are communicatively coupled via a local interface. The local interface can be, for example, but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the other computer components.

The processor(s) may be programmed to perform the functions of the method as described above. The processor(s) is a hardware device for executing software, particularly software stored in memory. Processor(s) can be any custom made or commercially available processor, a central processing unit (CPU), an auxiliary processor among several processors associated with a computer, a semiconductor based microprocessor (in the form of a microchip or chip set), a macroprocessor, or generally any device for executing software instructions.

Memory is associated with processor(s) and can include any one or a combination of volatile memory elements (e.g., random access memory (RAM, such as DRAM, SRAM, SDRAM, etc.)) and non-volatile memory elements (e.g., ROM, hard drive, tape, CDROM, etc.). Moreover, memory may incorporate electronic, magnetic, optical, and/or other types of storage media. Memory can have a distributed architecture where various components are situated remote from one another, but are still accessed by processor(s). The software in memory may include one or more separate programs. The separate programs comprise ordered listings of executable instructions for implementing logical functions in order to implement the functions of the modules. In the example of heretofore described, the software in memory includes the one or more components of the method and is executable on a suitable operating system (O/S).

The present teaching may include components provided as a source program, executable program (object code), script, or any other entity comprising a set of instructions to be performed. When a source program, the program needs to be translated via a compiler, assembler, interpreter, or the like, which may or may not be included within the memory, so as to operate properly in connection with the O/S. Furthermore, a methodology implemented according to the teaching may be expressed as (a) an object oriented programming language, which has classes of data and methods, or (b) a procedural programming language, which has routines, subroutines, and/or functions, for example but not limited to, C, C++, Pascal, Basic, Fortran, Cobol, Perl, Java, and Ada.

When the method is implemented in software, it should be noted that such software can be stored on any computer readable medium for use by or in connection with any computer related system or method. In the context of this teaching, a computer readable medium is an electronic, magnetic, optical, or other physical device or means that can contain or store a computer program for use by or in connection with a computer related system or method. Such an arrangement can be embodied in any computer readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be for example, but not limited to, an electronic, magnetic, optical, electromagnetic, infrared, or semiconductor system, apparatus, device, or propagation medium. Any process descriptions or blocks in the Figures, should be understood as representing modules, segments, or portions of code which include one or more executable instructions for implementing specific logical functions or steps in the process, as would be understood by those having ordinary skill in the art. The invention is not limited to the embodiment(s) described herein but can be amended or modified without departing from the scope of the present invention as defined by the claims.

## Claims

1. A visualization and drawing method for data representing a three dimensional (3D) scene, the method comprising:
rendering, on a display device, a two dimensional (2D) image of said 3D scene generated from said 3D scene data;
determining a 3D location of a pointer with respect to said 3D scene;
defining a highlight region in said 3D scene with respect to said 3D pointer location;
determining which of said 3D scene data is within said highlight region;
causing the 3D scene data within said highlight region to be highlighted when rendered on said display device in said 2D image,
generating 3D drawing structure data from at least said 3D pointer location, and
causing a 3D drawing structure corresponding to said 3D drawing structure data to be rendered in said 2D image, **characterised by**
incorporating said 3D drawing structure data into said 3D scene data.

2. The method of claim 1 including generating said 3D drawing structure data from a plurality of 3D pointer locations.

3. The method of claim 1 including causing any part of, or all of, said 3D drawing structure within said highlight region to be highlighted when rendered on said display device in said 2D image.

4. The method of claim 1 including defining a second highlight region in said 3D scene with respect to said 3D pointer location or with respect to said 3D drawing structure, and causing any part of said 3D drawing structure, and optionally any 3D scene data, within said second highlight region to be highlighted when rendered on said display device in said 2D image.

5. The method of any preceding claim including generating said 3D drawing structure data from at least two 3D pointer locations corresponding to movement of said pointer with respect to said 3D scene.

6. The method of claim 5, including defining a 3D line in said 3D scene from said 3D drawing structure data, and causing said 3D line to be rendered in said 2D image, wherein said 3D line may have a two dimensional transverse cross-section.

7. The method of any preceding claim including generating said 3D drawing structure data and causing a 3D drawing structure corresponding to said 3D drawing structure data to be rendered in said 2D image during movement of said pointer, the method further including causing said highlight region to track said movement of said pointer, and wherein said 3D drawing structure may comprise a 3D line and said method may include causing said highlight region to track said 3D line.

8. The method claim 7, including causing a second highlight region to track said movement of said pointer, and, wherein said 3D drawing structure may comprise a 3D line and said method may include causing said second highlight region to track said 3D line.

9. The method of any preceding claim, further including, in response to detecting a change in said 3D pointer location with respect to said 3D scene, determining a new 3D location of a pointer with respect to said 3D scene;
defining a new highlight region in said 3D scene with respect to said new 3D pointer location;
determining which of said 3D scene data is within said new highlight region; and
causing the 3D scene data within said highlight region to be highlighted when rendered on said display device in said 2D image.

10. The method of any preceding claim, including causing said highlight region to track said movement of said pointer.

11. The method of any preceding claim, including causing at least part, and preferably substantially all of, said highlight region to be in front of said 3D pointer location in the direction of movement of said pointer, and/or causing said highlight region to be elongate, for example ellipsoidal, and preferably aligned with a direction of movement of the pointer.

12. The method of any preceding claim including locating a 2D pointer on the rendered 2D image and calculating said 3D pointer location from the location of said 2D pointer and said 3D scene data, or including locating a 3D pointer in the rendered 3D scene.

13. The method of any preceding claim including analysing the 3D scene data with respect to one or more 3D pointer location, identifying one or more 3D structure in the relevant 3D scene data and generating 3D drawing structure data corresponding to the, or each, identified 3D structure.

14. The method of any preceding claim including defining a 3D selection structure from at least one of said 3D pointer locations and determining which of said 3D scene data corresponds with said 3D selection structure, and wherein, preferably, defining said 3D selection structure involves generating 3D selection structure data from said at least one 3D pointer location, and wherein the method preferably includes performing one or more processes on the 3D scene data corresponding to said 3D selection structure data, and wherein said 3D selection structure may be a 3D drawing structure.

15. A visualization system for visualizing data representing a three dimensional (3D) scene, the system comprising a display device and a computer-implemented 3D visualization module, said 3D visualization module being configured to cause a two dimensional (2D) image of said 3D scene generated from said 3D scene data to be displayed on said display device, to determine a 3D location of a pointer with respect to said 3D scene, to define a highlight region in said 3D scene with respect to said 3D pointer location, to cause the 3D scene data within said highlight region to be highlighted when rendered on said display device in said 2D image, to generate 3D drawing structure data from at least said 3D pointer location, and to cause a 3D drawing structure corresponding to said 3D drawing structure data to be rendered in said 2D image,
**characterised in that** said 3D visualization module being configured to incorporate said 3D drawing structure data into said 3D scene data.

## Patentansprüche

1. Visualisierungs- und Zeichnungsverfahren für Daten, die eine dreidimensionale (3D) Szene darstellen, wobei das Verfahren Folgendes beinhaltet:
Rendern, auf einem Anzeigegerät, eines zweidimensionalen (2D) Bildes der genannten, von den genannten 3D-Szenendaten erzeugten 3D-Szene;
Bestimmen eines 3D-Orts eines Zeigers mit Bezug auf die genannte 3D-Szene;
Definieren einer Highlight-Region in der genannten 3D-Szene mit Bezug auf den genannten 3D-Zeigerort;
Feststellen, welche der genannten 3D-Szenendaten innerhalb der genannten Highlight-Region liegen;
Bewirken, dass die 3D-Szenendaten in der genannten Highlight-Region hervorgehoben werden, wenn sie auf dem genannten Anzeigegerät in dem genannten 2D-Bild gerendert werden,
Erzeugen von 3D-Zeichnungsstrukturdaten von wenigstens dem genannten 3D-Zeigerort, und
Bewirken, dass eine 3D-Zeichnungsstruktur entsprechend den genannten 3D-Zeichnungsstrukturdaten in dem genannten 2D-Bild gerendert wird, **gekennzeichnet durch**
Integrieren der genannten 3D-Zeichnungsstrukturdaten in die genannten 3D-Szenendaten.

2. Verfahren nach Anspruch 1, das das Erzeugen der genannten 3D-Zeichnungsstrukturdaten von mehreren 3D-Zeigerorten beinhaltet.

3. Verfahren nach Anspruch 1, das das Bewirken beinhaltet, dass ein beliebiger Teil oder die Gesamtheit der genannten 3D-Zeichnungsstruktur in der genannten Highlight-Region hervorgehoben wird, wenn sie auf dem genannten Anzeigegerät in dem genannten 2D-Bild gerendert wird.

4. Verfahren nach Anspruch 1, das das Definieren einer zweiten Highlight-Region in der genannten 3D-Szene mit Bezug auf den genannten 3D-Zeigerort oder mit Bezug auf die genannte 3D-Zeichnungsstruktur und das Bewirken beinhaltet, dass ein beliebiger Teil der genannten 3D-Zeichnungsstruktur, und optional beliebige 3D-Szenendaten, innerhalb der genannten zweiten Highlight-Region hervorgehoben werden, wenn sie auf dem genannten Anzeigegerät in dem genannten 2D-Bild gerendert werden.

5. Verfahren nach einem vorherigen Anspruch, das das Erzeugen der genannten 3D-Zeichnungsstrukturdaten von wenigstens zwei 3D-Zeigerorten entsprechend einer Bewegung des genannten Zeigers mit Bezug auf die genannte 3D-Szene beinhaltet.

6. Verfahren nach Anspruch 5, das das Definieren einer 3D-Linie in der genannten 3D-Szene anhand der genannten 3D-Zeichnungsstrukturdaten und das Bewirken beinhaltet, dass die genannte 3D-Linie in dem genannten 2D-Bild gerendert wird, wobei die genannte 3D-Linie einen zweidimensionalen transversalen Querschnitt haben kann.

7. Verfahren nach einem vorherigen Anspruch, das das Erzeugen der genannten 3D-Zeichnungsstrukturdaten und das Bewirken beinhaltet, dass eine 3D-Zeichnungsstruktur entsprechend den genannten 3D-Zeichnungsstrukturdaten in dem genannten 2D-Bild während der Bewegung des genannten Zeigers gerendert wird, wobei das Verfahren ferner das Bewirken beinhaltet, dass die genannte Highlight-Region die genannte Bewegung des genannten Zeigers verfolgt, und wobei die genannte 3D-Zeichnungsstruktur eine 3D-Linie umfassen kann und das genannte Verfahren das Bewirken beinhalten kann, dass die genannte Highlight-Region die genannte 3D-Linie verfolgt.

8. Verfahren nach Anspruch 7, das das Bewirken beinhaltet, dass eine zweite Highlight-Region die genannte Bewegung des genannten Zeigers verfolgt, und wobei die genannte 3D-Zeichnungsstruktur eine 3D-Linie umfassen kann und das genannte Verfahren das Bewirken beinhalten kann, dass die genannte zweite Highlight-Region die genannte 3D-Linie verfolgt.

9. Verfahren nach einem vorherigen Anspruch, das ferner als Reaktion auf die Erkennung einer Änderung in dem genannten 3D-Zeigerort mit Bezug auf die genannte 3D-Szene das Feststellen eines neuen 3D-Orts eines Zeigers mit Bezug auf die genannte 3D-Szene beinhaltet;
Definieren einer neuen Highlight-Region in der genannten 3D-Szene mit Bezug auf den genannten neuen 3D-Zeigerort;
Feststellen, welche der genannten 3D-Szenendaten sich innerhalb der genannten neuen Highlight-Region befinden; und
Bewirken, dass die 3D-Szenendaten in der genannten Highlight-Region hervorgehoben werden, wenn sie auf dem genannten Anzeigegerät in dem genannten 2D-Bild gerendert werden.

10. Verfahren nach einem vorherigen Anspruch, das das Bewirken beinhaltet, dass die genannte Highlight-Region die genannte Bewegung des genannten Zeigers verfolgt.

11. Verfahren nach einem vorherigen Anspruch, das das Bewirken beinhaltet, dass sich wenigstens ein Teil, und vorzugsweise im Wesentlichen die Gesamtheit der genannten Highlight-Region vor dem genannten 3D-Zeigerort in der Bewegungsrichtung des genannten Zeigers befindet, und/oder das Bewirken beinhaltet, dass die genannte Highlight-Region länglich, zum Beispiel ellipsoid, und vorzugsweise mit einer Bewegungsrichtung des Zeigers ausgerichtet ist.

12. Verfahren nach einem vorherigen Anspruch, das das Positionieren eines 2D-Zeigers auf dem gerenderten 2D-Bild und das Berechnen des genannten 3D-Zeigerorts anhand des Orts des genannten 2D-Zeigers und der genannten 3D-Szenendaten oder das Positionieren eines 3D-Zeigers in der gerenderten 3D-Szene beinhaltet.

13. Verfahren nach einem vorherigen Anspruch, das das Analysieren der 3D-Szenendaten mit Bezug auf einen oder mehrere 3D-Zeigerorte, das Identifizieren von einer oder mehreren 3D-Strukturen in den relevanten 3D-Szenendaten und das Erzeugen von 3D-Zeichnungsstrukturdaten entsprechend der, oder jeder, identifizierten 3D-Struktur beinhaltet.

14. Verfahren nach einem vorherigen Anspruch, das das Definieren einer 3D-Auswahlstruktur von wenigstens einem der genannten 3D-Zeigerorte und das Bestimmen beinhaltet, welche der genannten 3D-Szenendaten der genannten 3D-Auswahlstruktur entsprechen, und wobei vorzugsweise das Definieren der genannten 3D-Auswahlstruktur das Erzeugen von 3D-Auswahlstrukturdaten von dem genannten wenigstens einen 3D-Zeigerort beinhaltet, und wobei das Verfahren vorzugsweise das Durchführen von einem oder mehreren Prozessen an den 3D-Szenendaten entsprechend den genannten 3D-Auswahlstrukturdaten beinhaltet, und wobei die genannte 3D-Auswahlstruktur eine 3D-Zeichnungsstruktur sein kann.

15. Visualisierungssystem zum Visualisieren von Daten, die eine dreidimensionale (3D) Szene darstellen, wobei das System ein Anzeigegerät und ein computerimplementiertes 3D-Visualisierungsmodul umfasst, wobei das genannte 3D-Visualisierungsmodul konfiguriert ist zum Bewirken, dass ein zweidimensionales (2D) Bild der genannten, von den genannten 3D-Szenendaten erzeugten 3D-Szene auf dem genannten Anzeigegerät angezeigt wird, zum Bestimmen eines 3D-Orts eines Zeigers mit Bezug auf die genannte 3D-Szene, zum Definieren einer Highlight-Region in der genannten 3D-Szene mit Bezug auf den genannten 3D-Zeigerort, zum Bewirken, dass die 3D-Szenendaten innerhalb der genannten Highlight-Region hervorgehoben werden, wenn sie auf dem genannten Anzeigegerät in dem genannten 2D-Bild gerendert werden, zum Erzeugen von 3D-Zeichnungsstrukturdaten von wenigstens dem genannten 3D-Zeigerort und zum Bewirken, dass eine 3D-Zeichnungsstruktur entsprechend den genannten 3D-Zeichnungsstrukturdaten in dem genannten 2D-Bild gerendert wird, **dadurch gekennzeichnet, dass** das genannte 3D-Visualisierungsmodul zum Integrieren der genannten 3D-Zeichnungsstrukturdaten in die genannten 3D-Szenendaten konfiguriert ist.

## Revendications

1. Procédé de visualisation et de dessin pour la représentation de données d'une scène tridimensionnelle (3D), le procédé comprenant les étapes consistant à :
restituer, sur un dispositif d'affichage, une image bidimensionnelle (2D) de ladite scène 3D générée à partir desdites données de scène 3D ;
déterminer une localisation 3D d'un pointeur par rapport à ladite scène 3D ;
définir une région de surbrillance dans ladite scène 3D par rapport à ladite localisation de pointeur 3D ;
déterminer, parmi lesdites données de scène 3D, celles qui sont dans ladite région de surbrillance ;
faire en sorte que les données de scène 3D dans ladite région de surbrillance soient mises en surbrillance lors de la restitution sur ledit dispositif d'affichage dans ladite image 2D ;
générer des données de structure de dessin 3D à partir, au moins, de ladite localisation de pointeur 3D ; et
faire en sorte qu'une structure de dessin 3D correspondant auxdites données de structure de dessin 3D soit restituée dans ladite image 2D,
le procédé étant **caractérisé par** l'étape consistant à :
intégrer lesdites données de structure de dessin 3D dans lesdites données de scène 3D.

2. Procédé selon la revendication 1, comprenant l'étape consistant à générer lesdites données de structure de dessin 3D à partir d'une pluralité de localisations de pointeur 3D.

3. Procédé selon la revendication 1, comprenant l'étape consistant à faire en sorte qu'une partie quelconque ou que la totalité de ladite structure de dessin 3D dans ladite région de surbrillance soit mise en surbrillance lors de la restitution sur ledit dispositif d'affichage dans ladite image 2D.

4. Procédé selon la revendication 1, comprenant l'étape consistant à définir une seconde région de surbrillance dans ladite scène 3D par rapport à ladite localisation de pointeur 3D ou par rapport à ladite structure de dessin 3D, et faire en sorte qu'une partie quelconque de ladite structure de dessin 3D, et éventuellement que des données quelconques de scène 3D, dans ladite seconde région de surbrillance soient mises en surbrillance lors de la restitution sur ledit dispositif d'affichage dans ladite image 2D.

5. Procédé selon l'une quelconque des revendications précédentes, comprenant l'étape consistant à générer lesdites données de structure de dessin 3D à partir d'au moins deux localisations de pointeur 3D correspondant à un déplacement dudit pointeur par rapport à ladite scène 3D.

6. Procédé selon la revendication 5, comprenant l'étape consistant à définir une ligne 3D dans ladite scène 3D à partir desdites données de structure de dessin 3D, et faire en sorte que ladite ligne 3D soit restituée dans ladite image 2D, ladite ligne 3D pouvant avoir une section transversale bidimensionnelle.

7. Procédé selon l'une quelconque des revendications précédentes, comprenant l'étape consistant à générer lesdites données de structure de dessin 3D et faire en sorte qu'une structure de dessin 3D correspondant auxdites données de structure de dessin 3D soit restituée dans ladite image 2D pendant le déplacement dudit pointeur, le procédé comprenant en outre l'étape consistant à faire en sorte que ladite région de surbrillance suive ledit déplacement dudit pointeur, ladite structure de dessin 3D pouvant comprendre une ligne 3D et ledit procédé pouvant comprendre l'étape consistant à faire en sorte que ladite région de surbrillance suive ladite ligne 3D.

8. Procédé selon la revendication 7, comprenant l'étape consistant à faire en sorte qu'une seconde région de surbrillance suive ledit déplacement dudit pointeur, ladite structure de dessin 3D pouvant comprendre une ligne 3D et ledit procédé pouvant comprendre l'étape consistant à faire en sorte que ladite seconde région de surbrillance suive ladite ligne 3D.

9. Procédé selon l'une quelconque des revendications précédentes, comprenant en outre, en réponse à la détection d'un changement de ladite localisation de pointeur 3D par rapport à ladite scène 3D, les étapes consistant à déterminer une nouvelle localisation 3D d'un pointeur par rapport à ladite scène 3D ;
définir une nouvelle région de surbrillance dans ladite scène 3D par rapport à ladite nouvelle localisation de pointeur 3D ;
déterminer, parmi lesdites données de scène 3D, celles qui sont dans ladite nouvelle région de surbrillance ; et
faire en sorte que les données de scène 3D dans ladite région de surbrillance soient mises en surbrillance lors de la restitution sur ledit dispositif d'affichage dans ladite image 2D.

10. Procédé selon l'une quelconque des revendications précédentes, comprenant l'étape consistant à faire en sorte que ladite région de surbrillance suive ledit déplacement dudit pointeur.

11. Procédé selon l'une quelconque des revendications précédentes, comprenant l'étape consistant à faire en sorte que ladite région de surbrillance soit, au moins en partie et de préférence entièrement, en face de ladite localisation de pointeur 3D dans la direction de déplacement dudit pointeur, et/ou faire en sorte que ladite région de surbrillance soit allongée, par exemple ellipsoïdale, et de préférence alignée avec une direction de déplacement du pointeur.

12. Procédé selon l'une quelconque des revendications précédentes, comprenant l'étape consistant à localiser un pointeur 2D sur l'image 2D restituée et calculer ladite localisation de pointeur 3D à partir de la localisation dudit pointeur 2D et desdites données de scène 3D, ou comprenant l'étape consistant à localiser un pointeur 3D dans la scène 3D restituée.

13. Procédé selon l'une quelconque des revendications précédentes, comprenant l'étape consistant à analyser les données de scène 3D par rapport à une ou plusieurs localisations de pointeur 3D, identifier une ou plusieurs structures 3D dans les données de scène 3D pertinentes et générer des données de structure de dessin 3D correspondant à la structure 3D identifiée ou à chacune des structures 3D identifiées.

14. Procédé selon l'une quelconque des revendications précédentes, comprenant l'étape consistant à définir une structure de sélection 3D à partir d'au moins une desdites localisations de pointeur 3D et déterminer, parmi lesdites données de scène 3D, celles qui correspondent à ladite structure de sélection 3D, la définition de ladite structure de sélection 3D impliquant, de préférence, la génération de données de structure de sélection 3D à partir de ladite au moins une localisation de pointeur 3D, et le procédé comprenant, de préférence, l'étape consistant à mettre en oeuvre un ou plusieurs processus sur les données de scène 3D correspondant auxdites données de structure de sélection 3D, ladite structure de sélection 3D pouvant être une structure de dessin 3D.

15. Système de visualisation pour visualiser des données représentant une scène tridimensionnelle (3D), le système comprenant un dispositif d'affichage et un module de visualisation 3D mis en oeuvre par ordinateur, ledit module de visualisation 3D étant configuré pour faire en sorte qu'une image bidimensionnelle (2D) de ladite scène 3D générée à partir desdites données de scène 3D soit affichée sur ledit dispositif d'affichage, pour déterminer une localisation 3D d'un pointeur par rapport à ladite scène 3D, pour définir une région de surbrillance dans ladite scène 3D par rapport à ladite localisation de pointeur 3D, pour faire en sorte que les données de scène 3D dans ladite région de surbrillance soient mises en surbrillance lors de la restitution sur ledit dispositif d'affichage dans ladite image 2D, pour générer des données de structure de dessin 3D à partir, au moins, de ladite localisation de pointeur 3D, et pour faire en sorte qu'une structure de dessin 3D correspondant auxdites données de structure de dessin 3D soit restituée dans ladite image 2D,
le système étant **caractérisé en ce que** ledit module de visualisation 3D est configuré pour intégrer lesdites données de structure de dessin 3D dans lesdites données de scène 3D.
